(19) Europäisches Patentamt
European Patent Office
Office européen des brevets

(11) EP 2 468 362 B1

(12) # EUROPÄISCHE PATENTSCHRIFT

(45) Veröffentlichungstag und Bekanntmachung des Hinweises auf die Patenterteilung:
**17.10.2018 Patentblatt 2018/42**

(51) Int Cl.:
*A61Q 17/04* (2006.01) *A61K 8/06* (2006.01)
*A61K 8/34* (2006.01) *A61K 8/35* (2006.01)
*A61K 8/41* (2006.01) *A61K 8/46* (2006.01)

(21) Anmeldenummer: **11191246.5**

(22) Anmeldetag: **30.11.2011**

(54) **Kosmetische Zubereitung mit UV-A-Filtersubstanz und Natriumcetearylsulfat**

Cosmetic preparation with a UVA-filter and sodium cetearyl sulfate

Préparation cosmétique comprenant un filtre UVA et du sulfate cétéarylique sodique

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2010 DE 102010063745**

(43) Veröffentlichungstag der Anmeldung:
**27.06.2012 Patentblatt 2012/26**

(73) Patentinhaber: **Beiersdorf AG**
**20253 Hamburg (DE)**

(72) Erfinder:
- **Nielsen, Jens**
  **22529 Hamburg (DE)**
- **Kröpke, Rainer**
  **22869 Schenefeld (DE)**
- **Kallmayer, Volker**
  **22393 Hamburg (DE)**
- **Morgenstern, Kirsten**
  **22303 Hamburg (DE)**
- **Lüttig, Kaja**
  **20255 Hamburg (DE)**

(56) Entgegenhaltungen:
**EP-A1- 1 078 638**
**EP-A1- 2 106 783**
**DE-A1-102007 005 186**
**DE-A1-102008 018 787**
**US-B2- 7 014 842**

- **ROLF DANIELS ET AL: "Galenics of dermal products ? vehicles, properties and drug release", JDDG: JOURNAL DER DEUTSCHEN DERMATOLOGISCHEN GESELLSCHAFT, Bd. 5, Nr. 5, 1. Mai 2007 (2007-05-01), Seiten 367-383, XP055199715, ISSN: 1610-0379, DOI: 10.1111/j.1610-0387.2007.06321.x**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 468 362 B1

## Beschreibung

Die vorliegende Erfindung betrifft eine kosmetische Zubereitung mit Natriumcetearylsulfat, mindestens eine UV-A-Filtersubstanz,sowie Ethylhexylglycerin und/oder 1,2-Pentandiol.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) bei übermäßiger Bestrahlung mit Licht aus dem UVB-Bereich (Wellenlänge: 280-320 nm) treten Langzeitschäden wie ein erhöhtes Risiko an Hautkrebs zu erkranken auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Darüber hinaus führt UV-Strahlung zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der EU-Kosmetikverordnung zusammengefasst. Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen. Beispielsweise stellt es ein Problem dar, UV-Filter-haltige (insbesondere UV-A-Filter-haltige) Zusammensetzungen herzustellen, die einerseits wasserfest sind und eine hautbefeuchtende Leistung aufweisen und auf der anderen Seite ein angenehmes, nicht klebriges Hautgefühl besitzen. Denn zum einen wird zur Hautbefeuchtung in der Regel das relativ klebrige Glycerin verwendet; zur Erhöhung der Wasserfestigkeit finden in herkömmlichen Produkten in der Regel polymere Filmbildner Verwendung, die ebenfalls die Klebrigkeit erhöhen. Klebrige Sonnenschutzprodukte führen, neben einer unangenehmen Sensorik, in der Regel auch dazu, dass, beispielsweise bei Verwendung am Strand, die Anhaftung von Fremdpartikeln wie Sand gefördert wird, was ebenfalls unerwünscht ist.

Ein weiterer Nachteil des Standes der Technik besteht darin, dass Zubereitungen mit einem hohen UVA-Schutz relativ hohe Mengen an UVA-Filtern enthalten müssen. Die üblicherweise eingesetzten UVA-Filter sind jedoch feste Stoffe, die durch Lipide gelöst bzw. in Lösung gehalten werden müssen, damit sie ihre Wirkung nicht verlieren. Diese Lipide wirken dann später oft fettig und glänzend auf der Haut.

Generell stellt es einen Mangel des Standes der Technik dar, dass erhöhte Anteile an UV-Filtersubstanzen in kosmetischen Zubereitungen zu hautunfreundlicheren Zubereitungen führen, die sich stumpf und klebrig anfühlen, schwer verreibbar sind, problematischer zu formulieren und nicht langzeitstabil sind. Wünschenswert ist es daher, Zubereitungen zur Verfügung zu stellen, die bei gleichbleibendem UV-Filtergehalt eine verbesserte Lichtschutzleistung erbringen ohne dass Nachteile in anderen Wirkungen der Zubereitungen damit einhergehen.

Es war daher die Aufgabe der vorliegenden Erfindung, die Mängel des Standes der Technik zu beseitigen und eine kosmetische Zubereitung zu entwickeln, die einerseits eine hohe hautbefeuchtende Leistung aufweist und wasserfest ist und auf der anderen Seite eine angenehme Sensorik auf der Haut hinterlässt. Die Zubereitungen sollten auf der Haut nicht klebrig wirken und die Neigung der Anhaftung von Fremdpartikeln, wie Sand, auf der Haut sollte deutlich reduziert werden.

Überraschend gelöst wird die Aufgabe durch eine kosmetische Zubereitung enthaltend

a) Natriumcetearylsulfat,
b) mindestens eine UV-A-Filtersubstanz und
c) Ethylhexylglycerin, wobei die Zubereitung frei ist von Konservierungsmitteln oder 1,2-Pentandiol enthält,
oder die Zubereitung
d) 1,2-Pentandiol enthält, wobei die Zubereitung frei ist von Polyethylenglycol, Polyethylenglycolderivaten und Konservierungssmitteln.

Zwar kennt der Fachmann die DE 10361202, DE 102006061689, EP 1837055 und WO 2005077327, doch konnten dieser Stand der Technik nicht den Weg zur vorliegenden Erfindung weisen. Darüber hinaus kennt der Fachmann die DE 102008018787 A1, EP 2106783 A1, DE 102007005186 A1, US 7014842 B2, EP 1078638 A1 und den Artikel "Galenic of dermal products-vehicles, properties and drug release" von R. Daniels und U. Knie, JDDG, 2007, 5, Seiten 367-383, die ebenfalls nicht den Weg zur vorliegenden Erfindung weisen konnten.

Es ist erfindungsgemäß vorteilhaft, wenn die Zubereitung Natriumcetearylsulfat in einer Konzentration von 0,05 bis 0,5 Gew.-%, bezogen auf das Gesamtgewicht der Zubereitung, enthält.

Es ist erfindungsgemäß bevorzugt, wenn die Zubereitung Natriumcetearylsulfat in einer Konzentration von 0,1 bis 0,3 Gew.-%, bezogen auf das Gesamtgewicht der Zubereitung, enthält.

Es ist erfindungsgemäß vorteilhaft, wenn die erfindungsgemäße Zubereitung frei ist von Polyethylenglycol und Polyethylenglycolderivaten. Unter Polyethylenglycolderivaten werden dabei erfindungsgemäß Verbindungen verstanden, die eine oder mehrere Polyethylenglycol-Ketten enthalten.

Polyethylenglycol und Polyethylenglycolderivate werden üblicherweise in kosmetischen Zubereitungen dazu verwendet, die hautbefeuchtende Leistung der Zubereitung weiter zu erhöhen und, im Falle dass die Zubereitung in Form einer Emulsion vorliegt, die Stabilität der Emulsion zu verbessern (= zu erhöhen). Es war daher umso überraschender, dass die PEG-freien Zubereitungen eine gleichwertige Hautbefeuchtung und Stabilität aufweisen.

Es ist erfindungsgemäß von Vorteil, wenn die erfindungsgemäße Zubereitung frei ist von Konservierungsstoffen. Dabei werden als Konservierungsstoffe im Rahmen der vorliegenden Offenbarung die folgenden Verbindungen angesehen: Formaldehydabspalter (wie z. B. DMDM Hydantoin, welches beispielsweise unter der Handelsbezeichnung Glydant™ von der Fa. Lonza erhältlich ist), Iodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol.

Erfindungsgemäß vorteilhafte Ausführungsformen der vorliegenden Erfindung sind dadurch gekennzeichnet, dass die Zubereitung Polyglyceryl-2 Caprate, Glycerylcaprylate und/oder Phenyethylethanol enthält.

Polyglyceryl-2 Caprate werden dabei erfindungsgemäß bevorzugt in einer Konzentration von 0,05 bis 5,0 Gew.-%, bezogen auf das Gesamtgewicht der Zubereitung eingesetzt, Glycerylcaprylate werden erfindungsgemäß bevorzugt in einer Konzentration von 0,05 bis 5,0 Gew.-%, bezogen auf das Gesamtgewicht der Zubereitung eingesetzt, und Phenyethylethanol wird erfindungsgemäß bevorzugt in einer Konzentration von 0,15 bis 0,75 Gew.-%, bezogen auf das Gesamtgewicht der Zubereitung eingesetzt.

Es ist erfindungsgemäß vorteilhaft, wenn als UV-A-Filter 4-(tert.-Butyl)-4'-methoxydibenzoylmethan und/oder 2-(4'-Diethylamino-2'-hydoxybenzoyl)-benzoesäurehexylester eingesetzt werden.

Enthält die erfindungsgemäße Zubereitung 4-(tert.-Butyl)-4'-methoxydibenzoylmethan als erfindungsgemäßen UV-A-Filter, so ist es erfindungsgemäß vorteilhaft, wenn die Zubereitung 4-(tert.-Butyl)-4'-methoxydibenzoylmethan in einer Konzentration von 1,5 bis 15,0 Gew.-%, bezogen auf das Gesamtgewicht der Zubereitung, enthält. Erfindungsgemäß bevorzugt ist eine Einsatzkonzentration von 2,0 bis 5,5 Gew.-%, bezogen auf das Gesamtgewicht der Zubereitung.

Enthält die erfindungsgemäße Zubereitung 2-(4'-Diethylamino-2'-hydoxybenzoyl)-benzoesäurehexylester als erfindungsgemäßen UV-A-Filter, so ist es erfindungsgemäß vorteilhaft, wenn die Zubereitung 2-(4'-Diethylamino-2'-hydoxybenzoyl)-benzoesäurehexylester in einer Konzentration von 1,5 bis 15,0 Gew.-%, bezogen auf das Gesamtgewicht der Zubereitung, enthält. Erfindungsgemäß bevorzugt ist eine Einsatzkonzentration von 2,0 bis 5,5 Gew.-%, bezogen auf das Gesamtgewicht der Zubereitung.

Es ist erfindungsgemäß vorteilhaft, wenn die Zubereitung Ethylhexylglycerin in einer Konzentration von 0,005 bis 5,0 Gew.-%, bezogen auf das Gesamtgewicht der Zubereitung, enthält.

Es ist erfindungsgemäß bevorzugt, wenn die Zubereitung Ethylhexylglycerin in einer Konzentration von 0,5 bis 1,0 Gew.-%, bezogen auf das Gesamtgewicht der Zubereitung, enthält.

Es ist erfindungsgemäß vorteilhaft, wenn die Zubereitung 1,2-Pentandiol (auch Pentan-1,2-diol genannt) in einer Konzentration von 0,005 bis 5,0 Gew.-%, bezogen auf das Gesamtgewicht der Zubereitung, enthält.

Es ist erfindungsgemäß bevorzugt, wenn die Zubereitung 1,2-Pentandiol in einer Konzentration von 0,5 bis 2,0 Gew.-%, bezogen auf das Gesamtgewicht der Zubereitung, enthält.

Erfindungsgemäß vorteilhafte Ausführungsformen der vorliegenden Erfindung sind darüber hinaus dadurch gekennzeichnet, dass die Zubereitung Cetearylalkohol enthält.

In einem solchen Falle sind die erfindungsgemäß vorteilhaften Ausführungsformen dadurch gekennzeichnet, dass die Zubereitung Cetearylalkohol in einer Konzentration von 0,1 bis 10 Gew.-%, bezogen auf das Gesamtgewicht der Zubereitung, enthält. Erfindungsgemäß bevorzugt ist eine Einsatzkonzentration von 0,1 bis 3,5 Gew.-%, bezogen auf das Gesamtgewicht der Zubereitung.

Durch den Zusatz von Cetearylalkohol kann überraschenderweise auch die Viskosität der Zubereitung von sprühbar bis cremeförmig eingestellt werden, ohne dass an der Rezepturzusammensetzung hinsichtlich der Rohstoffe und Einsatzkonzentrationen große Änderungen vorgenommen werden müssten.

Erfindungsgemäß vorteilhaft liegt die erfindungsgemäße Zubereitung in Form einer Emulsion vor, wobei es erfindungsgemäß bevorzugt ist, wenn die Zubereitung in Form einer O/W-Emulsion vorliegt.

Es ist erfindungsgemäß vorteilhaft, wenn die erfindungsgemäße Zubereitung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; 2-Ethylhexyl-2-cyano-3,3-diphenylacrylat (Octocrylen); Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'-Dineopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate enthält.

Die erfindungsgemäß vorteilhaften Merocyanine werden dabei gewählt aus der Gruppe der Verbindungen

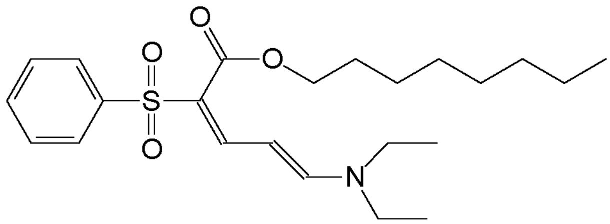

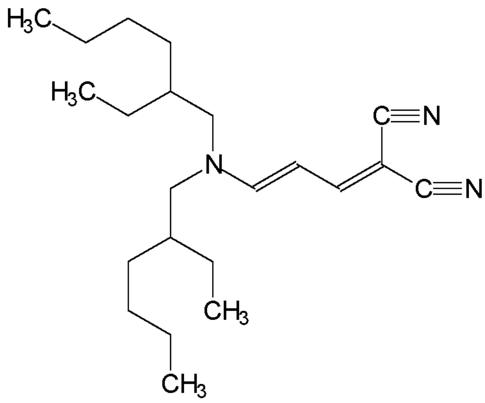

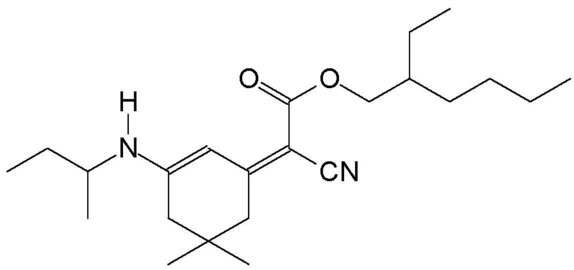

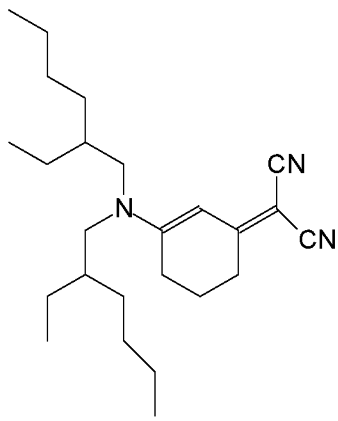

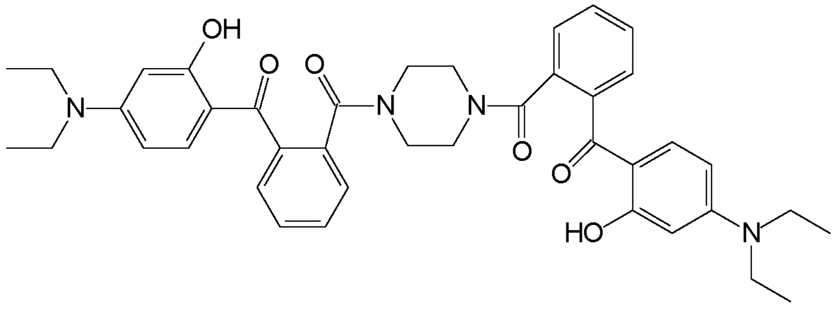

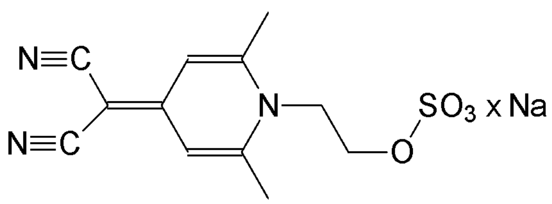

Als erfindungsgemäß vorteilhaftes Piperazinderivat kann dabei die folgende Verbindung eingesetzt werden:

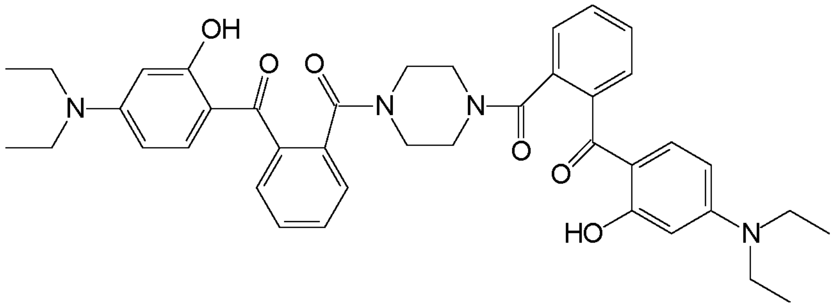

Es ist erfindungsgemäß vorteilhaft, wenn die erfindungsgemäße Zubereitung frei ist von p-Methylbenzylidencampher.
Es ist erfindungsgemäß vorteilhaft, wenn die erfindungsgemäße Zubereitung einen oder mehrere Parfümstoffe enthält.
Erfindungsgemäß bevorzugte Ausführungsformen im Sinne der vorliegenden Erfindung sind dadurch gekennzeichnet, dass die Zubereitung einen oder mehrere Parfümstoffe gewählt aus der Gruppe der Verbindungen Linalool, Benzyl Benzoate, Hydroxyisohexyl 3-Cyclohexene Carboxaldehyd, Hexyl Cinnamal, Benzyl Salicylate, Citronellol, Coumarin, enthält.
Es ist erfindungsgemäß vorteilhaft, wenn die Gesamtkonzentration an Parfümstoffen in der Zubereitung von 0,00001 bis 1,8 Gew.-%, bezogen auf das Gesamtgewicht der Zubereitung, beträgt.
Erfindungsgemäß vorteilhafte Ausführungsformen der vorliegenden Erfindung sind dadurch gekennzeichnet, dass die erfindungsgemäße Zubereitung ein oder mehrere Verbindungen gewählt aus der Gruppe der Alkohole, Glycole, Glycerin enthält.
Es ist erfindungsgemäß vorteilhaft, wenn die erfindungsgemäße Zubereitung Feuchthaltemittel wie Glycerin, Milchsäure und/oder Lactate, insbesondere Natriumlactat, Butylenglykol, Propylenglykol, Biosaccaride Gum-1, Glycine Soja, Glucosylglyceride, Pyrrolidoncarbonsäure, Hyaluronsäure, Chitosan und/oder Harnstoff enthält.
Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum.
Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner.
Erfindungsgemäß vorteilhafte Ausführungsformen der vorliegenden Erfindung sind dadurch gekennzeichnet, dass die Zubereitung als Verdickungsmittel eine oder mehrere Verbindungen gewählt aus der Gruppe der Verbindungen Acrylat/$C_{10}$-$C_{30}$-Alkylacrylat, Xanthangummi, Tapiokastärke enthält.
Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.
Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).
Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge

von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, $C_{12-13}$-Alkyllactat, Di-$C_{12-13}$-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an $C_{12-15}$-Alkylbenzoat in Kombination mit Benzoesäure aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Es ist erfindungsgemäß bevorzugt, wenn die erfindungsgemäße Zubereitung C12-15 Alkylbenzoat in Kombination mit Benzoesäure enthält.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:

(a) Siloxanelastomere, welche die Einheiten $R_2SiO$ und $RSiO_{1,5}$ und/oder $R_3SiO_{0,5}$ und/oder $SiO_2$ enthalten, wobei die einzelnen Reste R jeweils unabhängig voneinander Wasserstoff, $C_{1-24}$-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten $R_2SiO$ zu $RSiO_{1,5}$ aus dem Bereich von 1 : 1 bis 30 : 1 gewählt wird;

(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,

wobei die verwendeten Mengenateile so gewählt werden, dass die Menge des Wasserstoffes des Organopolysiloxans (1) oder der ungesättigten aliphatischen Gruppen des Organopolysiloxans (2)

- im Bereich von 1 bis 20 mol-% liegt, wenn das Organopolysiloxan nicht zyklisch ist und
- im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Vorteilhaft ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Erfindungsgemäß besonders bevorzugte Ausführungsformen der vorliegenden Erfindung sind dadurch gekennzeichnet, dass die Zubereitung eine oder mehrere lipophile Bestandteile gewählt aus der Gruppe der Verbindungen Myristylmyristat, Octyldodecanol, $C_{12}$-$C_{15}$-Alkylbenzoat + Benzoesäure, Butylenglycoldicaprylat/Dicaprat, Cetylalkohol enthält.

Weitere vorteilhafte Wirkstoffe im Sinne der vorliegenden Erfindung sind natürliche Wirkstoffe und/oder deren Derivate, wie z. B. alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Tocopherol und seine Derivate (beispielsweise das Acetat oder Palmitat), Vitamin A und seine Derivate, Gylcyrrhetinsäure, Kreatin, Kreatinin, Taurin und/oder β-Alanin sowie Licochalcon A.

Vorteilhaft im Sinne der vorliegenden Erfindung sind Zubereitungen zur Pflege der Haut. Sie können dem kosmetischen Lichtschutz, der Hautpflege (beispielsweise zur Prophylaxe und Behandlung der Hautalterung) und dekorativen Kosmetik dienen.

Die Zubereitungen können sprühbar oder auf einem Träger (z.B. Tuch) dargereicht werden.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

## Vergleichsversuch

Der erfinderische Effekt konnte mit dem folgenden Vergleichsversuch belegt werden:

Die Wirksamkeit von Sonnenschutzmittel bzw. der ihnen zugrunde liegenden UV-Filter wird in der Regel in biologischen Wirksamkeitsprüfungen unter standardisierten Bedingungen bestimmt.

Der Lichtschutzfaktor (LSF, oft auch SPF (sun protection factor) genannt) gibt die Verlängerung der Sonnenbestrahlung an, die durch Verwendung des Sonnenschutzmittels ermöglicht wird. Er ist der Quotient aus Erythemschwellenzeit mit Sonnenschutzmittel und Erythemschwellenzeit ohne Sonnenschutzmittel.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wie viel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muss die Zubereitung mindestens 90 % der UV-A-Strahlung im Bereich 320 -360 nm absorbieren.

UV-Strahlung verschiedener Frequenz/Wellenlänge dringt unterschiedlich tief ins Gewebe ein. Daher ist auch die Art der Schädigung wellenlängenabhängig. Dem UV-A-Schutz kommt hier eine besondere Bedeutung zu, da diese UV-Strahlung (UV-A (Wellenlänge 315 - 400 nm), im Wesentlichen für die Hautalterung verantwortlich ist

UVA-Filter zählen zu den chemischen Lichtschutzfiltern. Sie dringen in die Haut ein, nehmen dort die energiereiche UV-Strahlung auf und wandeln diese in Wärme- oder Lichtenergie um, welche keine Schäden mehr anrichten kann. Obwohl es für die Bestimmung der Schutzwirkung im UVA-Bereich aktuell kein anerkanntes Prüfverfahren gibt (im Gegensatz zu der COLIPA-Methode für UVB-Filter), sollte in der Praxis die so genannte UVA/UVB-Schutzbalance gelten. Das bedeutet, dass UVA-Filter in Relation von 1:3 zu UVB-Filtern eingesetzt werden und damit der UVA-Schutz mit steigendem Lichtschutz angehoben wird. Die EU-Empfehlung sieht einen UVA-Schutz von 1/3 im Verhältnis zum UVB-Schutz vor. Mit der neuen EU-Empfehlung soll demnächst jedes Sonnenschutzmittel gleichzeitig auch vor UVA-Strahlung schützen. Der Verbraucher soll in Zukunft davon ausgehen, dass ein UVA-Schutz gewährleistet ist, der mit steigendem SPF-Wert ansteigt. Aus ethischen Gründen schlägt sie dazu eine in-vitro-Methode vor. Diese Messmethode trägt den Namen "COLIPA Ratio". Die COLIPA Ratio muss der Formel <3 sein.

$$\frac{UVB}{UVA} < 3$$

Die COLIPA Ratio ist der Standard zur Bestimmung des UVA-Schutzes.

Erfindungsgemäß wird als Verbesserung der Lichtschutzleistung die Steigerung der in-vitro SPF, UVA-SPF und/oder Colipa Ratio Messwerte verstanden.

Es wurden folgende Zubereitung A und B hergestellt und deren in-vitro SPF, UVA-SPF und Colipa Ratio gemessen bzw. bestimmt. Zubereitung A ist eine Zubereitung mit entsprechenden UV Filtern aber ohne den erfindungsgemäßen SPF-Booster Ethylhexylglycerin, B ist die gleiche Zubereitung mit SPF Booster. Die Anteilsunterschiede werden jeweils durch den Anteil Wasser ausgeglichen.

| INCI/Gew.% | A ohne Ethylhexylglycerin | B mit Ethylhexylglycerin |
|---|---|---|
| Methylparaben | 0,3 | 0,3 |
| Trisodium EDTA | 1 | 1 |
| Ethylhexyl Methoxycinnamate | 0,5 | 0,5 |

(fortgesetzt)

| INCI/Gew.% | A ohne Ethylhexylglycerin | B mit Ethylhexylglycerin |
|---|---|---|
| Phenoxyethanol | 0,6 | 0,6 |
| Dimethicone | 2 | 2 |
| Hydrogenated Coco-Glycerides | 3 | 3 |
| C12-15 Alkyl Benzoate | 6 | 6 |
| Butyl Methoxydibenzoylmethane | 4,5 | 4,5 |
| Glycerin | 4,3 | 4,3 |
| Sodium Hydroxide | 0,185 | 0,185 |
| Alcohol Denat. | 4 | 4 |
| Xanthan Gum | 0,4 | 0,4 |
| Natriumcetearylsulfat | 0,1 | 0,1 |
| Cetearylalkohol | 3 | 3 |
| Ethylhexylglycerin | --- | 2,5 |
| Bis-Ethylhexyloxyphenol Methoxyphenyl Triazine | 1,5 | 1,5 |
| Butylene Glycol Dicaprylate/Dicaprate | 2 | 2 |
| Aqua | 66,515 | 64,015 |
| Colipa-Ratio (kleiner 3 = sehr gut; je niedriger desto besser) | 2,3 | 2,2 |
| in-vitro-SPF | 78,1 | 112,9 (+44%) |
| UVA-SPF (Spalte UVAPF Colipa nach Bestrahlung) | 12,3 | 13,7 |

Die Messungen zu in-vitro-SPF wurden entsprechend den Vorgaben gemäß "METHOD FOR THE IN VITRO DETERMINATION OF UVA PROTECTION PROVIDED BY SUNSCREEN PRODUCTS" Guideline 2007a, Prepared by the COLIPA *In vitro* Photoprotection Methods Task Force, durchgeführt.

Ein Colipa-Ratio von weniger als 2,3 bedeutet gegenüber der Vergleichszubereitung A eine Steigerung der Lichtschutzleistung.

In gleicher Weise ist eine Steigerung der in-vitro Lichtschutzleistung festgestellt worden durch in-vitro SPF Messwerte größer 78,1 sowie einem gesteigertem UVA SPF bei Werten größer 13,7.

Erfindungsgemäß führen die Verbindungen zu einem SPF-Boosting (Steigerung der Lichtschutzleistung) um 44%.

## Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### O/W-Emulsion

| | **1** | **2** | **3** | **4** | **5** |
|---|---|---|---|---|---|
| Glycerylsterat | 1,0 | 2,75 | 2,0 | 3,5 | 2,5 |
| Cetearylsulfat, Natrium-Salz | 0,1 | 0,2 | 0,12 | 0,14 | 0,075 |
| Cetearylalkohol | 0,5 | 1,5 | 1,618 | 0,14 | 0,75 |
| Silikonöl, linear | 0,5 | 1,3 | 1,5 | 1,2 | 1,75 |
| Butyl Methoxydibenzoylmethan | 3,5 | 4,5 | --- | 2,5 | 1,5 |

(fortgesetzt)

| | 1 | 2 | 3 | 4 | 5 |
|---|---|---|---|---|---|
| Ethylhexylmethoxycinnamat | 3,5 | 9,0 | 11,0 | 5,0 | 4,5 |
| 2-(4'-Diethylamino-2'-hydoxybenzoyl)-benzoesäurehexylester | 1,5 | --- | 5,5 | --- | 0,5 |
| Parfum | q,s, | q,s, | q,s, | q,s, | q,s, |
| Polyacrylsäure, Natrium-Salz | 0,4 | 0,1 | 0,15 | 0,05 | 0,1 |
| Ethylhexylglycerin | 0,3 | 2,5 | 3,75 | 0,15 | 1,5 |
| Glycerin | 5 | 10 | 10 | 15 | 7,5 |
| Wasser | ad 100 | ad 100 | ad 100 | ad 100 | ad 100 |

**Patentansprüche**

**1.** Kosmetische Zubereitung enthaltend

a) Natriumcetearylsulfat,
b) mindestens eine UV-A-Filtersubstanz und
c) Ethylhexylglycerin, wobei die Zubereitung frei ist von Konservierungsmitteln oder 1,2-Pentandiol enthält, oder die Zubereitung
c) 1,2-Pentandiol enthält, wobei die Zubereitung frei ist von Polyethylenglycol, Polyethylenglycolderivaten und Konservierungssmitteln.

**2.** Kosmetische Zubereitung nach Anspruch 1, **dadurch gekennzeichnet, dass** die Zubereitung frei ist von Polyethylenglycol und Polyethylenglycolderivaten.

**3.** Kosmetische Zubereitung nach einem der vorhergehenden Ansprüche, **dadurch gekennzeichnet, dass** die Zubereitung Polyglyceryl-2 Caprate und/oder Glycerylcaprylate enthält.

**4.** Kosmetische Zubereitung nach einem der vorhergehenden Ansprüche, **dadurch gekennzeichnet, dass** als UV-A-Filter 4-(tert.-Butyl)-4'-methoxydibenzoylmethan und/oder 2-(4'-Diethylamino-2'-hydroxybenzoyl)-benzoesäurehexylester eingesetzt werden.

**5.** Kosmetische Zubereitung nach einem der vorhergehenden Ansprüche, **dadurch gekennzeichnet, dass** die Zubereitung Natriumcetearylsulfat in einer Konzentration von 0,05 bis 0,5 Gew.-%, bezogen auf das Gesamtgewicht der Zubereitung, enthält.

**6.** Kosmetische Zubereitung nach einem der vorhergehenden Ansprüche, **dadurch gekennzeichnet, dass** die Zubereitung 4-(tert.-Butyl)-4'-methoxydibenzoylmethan in einer Konzentration von 1,5 bis 15,0 Gew.-%, bezogen auf das Gesamtgewicht der Zubereitung, enthält.

**7.** Kosmetische Zubereitung nach einem der vorhergehenden Ansprüche, **dadurch gekennzeichnet, dass** die Zubereitung 2-(4'-Diethylamino-2'-hydroxybenzoyl)-benzoesäurehexylester in einer Konzentration von 1,5 bis 15,0 Gew.-%, bezogen auf das Gesamtgewicht der Zubereitung, enthält.

**8.** Kosmetische Zubereitung nach einem der vorhergehenden Ansprüche, **dadurch gekennzeichnet, dass** die Zubereitung Ethylhexylglycerin in einer Konzentration von 0,005 bis 5,0 Gew.-%, bezogen auf das Gesamtgewicht der Zubereitung, enthält.

**9.** Kosmetische Zubereitung nach einem der vorhergehenden Ansprüche, **dadurch gekennzeichnet, dass** die Zubereitung 1,2-Pentandiol in einer Konzentration von 0,005 bis 5,0 Gew.-%, bezogen auf das Gesamtgewicht der Zubereitung, enthält.

**10.** Kosmetische Zubereitung nach einem der vorhergehenden Ansprüche, **dadurch gekennzeichnet, dass** die Zubereitung Cetearylalkohol enthält.

11. Kosmetische Zubereitung nach einem der vorhergehenden Ansprüche, **dadurch gekennzeichnet, dass** die Zubereitung Cetearylalkohol in einer Konzentration 0,1 von bis 10 Gew.-%, bezogen auf das Gesamtgewicht der Zubereitung, enthält.

12. Kosmetische Zubereitung nach einem der vorhergehenden Ansprüche, **dadurch gekennzeichnet, dass** die Zubereitung in Form einer O/W-Emulsion vorliegt.

## Claims

1. Cosmetic preparation comprising

   a) sodium cetearyl sulfate,
   b) at least one UVA filter substance and
   c) ethylhexylglycerin, wherein the preparation is free of preservatives or comprises 1,2-pentanediol,
   or the preparation comprises
   c) 1,2-pentanediol, wherein the preparation is free of polyethylene glycol, polyethylene glycol derivatives and preservatives.

2. Cosmetic preparation according to Claim 1, **characterized in that** the preparation is free of polyethylene glycol and polyethylene glycol derivatives.

3. Cosmetic preparation according to either of the preceding claims, **characterized in that** the preparation comprises polyglyceryl-2 caprate and/or glyceryl caprylate.

4. Cosmetic preparation according to any of the preceding claims, **characterized in that** 4-(tert-butyl)-4'-methoxydibenzoylmethane and/or hexyl 2-(4'-diethylamino-2'-hydroxybenzoyl)benzoate are used as UVA filter.

5. Cosmetic preparation according to any of the preceding claims, **characterized in that** the preparation comprises sodium cetearyl sulfate at a concentration of 0.05 to 0.5% by weight, based on the total weight of the preparation.

6. Cosmetic preparation according to any of the preceding claims, **characterized in that** the preparation comprises 4-(tert-butyl)-4'-methoxydibenzoylmethane at a concentration of 1.5 to 15.0% by weight, based on the total weight of the preparation.

7. Cosmetic preparation according to any of the preceding claims, **characterized in that** the preparation comprises hexyl 2-(4'-diethylamino-2'-hydroxybenzoyl)benzoate at a concentration of 1.5 to 15.0% by weight, based on the total weight of the preparation.

8. Cosmetic preparation according to any of the preceding claims, **characterized in that** the preparation comprises ethylhexylglycerin at a concentration of 0.005 to 5.0% by weight, based on the total weight of the preparation.

9. Cosmetic preparation according to any of the preceding claims, **characterized in that** the preparation comprises 1,2-pentanediol at a concentration of 0.005 to 5.0% by weight, based on the total weight of the preparation.

10. Cosmetic preparation according to any of the preceding claims, **characterized in that** the preparation comprises cetearyl alcohol.

11. Cosmetic preparation according to any of the preceding claims, **characterized in that** the preparation comprises cetearyl alcohol at a concentration of 0.1 to 10% by weight, based on the total weight of the preparation.

12. Cosmetic preparation according to any of the preceding claims, **characterized in that** the preparation is in the form of an O/W emulsion.

## Revendications

1. Préparation cosmétique, contenant :

a) du cétéarylsulfate de sodium,
b) au moins une substance de filtration des UV-A et
c) de l'éthylhexylglycérine, la préparation étant exempte de conservateurs ou contenant du 1,2-pentanediol, ou la préparation
c) contenant du 1,2-pentanediol, la préparation étant exempte de polyéthylène glycol, de dérivés de polyéthylène glycol et de conservateurs.

2. Préparation cosmétique selon la revendication 1, **caractérisée en ce que** la préparation est exempte de polyéthylène glycol et de dérivés de polyéthylène glycol.

3. Préparation cosmétique selon l'une quelconque des revendications précédentes, **caractérisée en ce que** la préparation contient du 2-caprate de polyglycéryle et/ou du caprylate de glycéryle.

4. Préparation cosmétique selon l'une quelconque des revendications précédentes, **caractérisée en ce que** du 4-(tert.-butyl)-4'-méthoxydibenzoylméthane et/ou de l'ester hexylique de l'acide 2-(4'-diéthylamino-2'-hydroxybenzoyl)-benzoïque sont utilisés en tant que filtres UV-A.

5. Préparation cosmétique selon l'une quelconque des revendications précédentes, **caractérisée en ce que** la préparation contient du cétéarylsulfate de sodium en une concentration de 0,05 à 0,5 % en poids, par rapport au poids total de la préparation.

6. Préparation cosmétique selon l'une quelconque des revendications précédentes, **caractérisée en ce que** la préparation contient du 4-(tert.-butyl)-4'-méthoxydibenzoylméthane en une concentration de 1,5 à 15,0 % en poids, par rapport au poids total de la préparation.

7. Préparation cosmétique selon l'une quelconque des revendications précédentes, **caractérisée en ce que** la préparation contient de l'ester hexylique de l'acide 2-(4'-diéthylamino-2'-hydroxybenzoyl)-benzoïque en une concentration de 1,5 à 15,0 % en poids, par rapport au poids total de la préparation.

8. Préparation cosmétique selon l'une quelconque des revendications précédentes, **caractérisée en ce que** la préparation contient de l'éthylhexylglycérine en une concentration de 0,005 à 5,0 % en poids, par rapport au poids total de la préparation.

9. Préparation cosmétique selon l'une quelconque des revendications précédentes, **caractérisée en ce que** la préparation contient du 1,2-pentanediol en une concentration de 0,005 à 5,0 % en poids, par rapport au poids total de la préparation.

10. Préparation cosmétique selon l'une quelconque des revendications précédentes, **caractérisée en ce que** la préparation contient de l'alcool cétéarylique.

11. Préparation cosmétique selon l'une quelconque des revendications précédentes, **caractérisée en ce que** la préparation contient de l'alcool cétéarylique en une concentration de 0,1 à 10 % en poids, par rapport au poids total de la préparation.

12. Préparation cosmétique selon l'une quelconque des revendications précédentes, **caractérisée en ce que** la préparation se présente sous la forme d'une émulsion H/E.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10361202
- DE 102006061689
- EP 1837055 A
- WO 2005077327 A
- DE 102008018787 A1
- EP 2106783 A1
- DE 102007005186 A1
- US 7014842 B2
- EP 1078638 A1


### In der Beschreibung aufgeführte Nicht-Patentliteratur


- **VON R. DANIELS ; U. KNIE.** Galenic of dermal products-vehicles, properties and drug release. *JDDG,* 2007, vol. 5, 367-383
- *CHEMICAL ABSTRACTS,* 288254-16-0
- METHOD FOR THE IN VITRO DETERMINATION OF UVA PROTECTION PROVIDED BY SUNSCREEN PRODUCTS. *Guideline,* 2007